# EUROPÄISCHE PATENTSCHRIFT

(11) **EP 0 891 971 B1**
(45) Veröffentlichungstag und Bekanntmachung des Hinweises auf die Patenterteilung: **12.11.2003**
(21) Anmeldenummer: 98110632.1
(22) Anmeldetag: 10.06.1998
(51) Int. Cl.: C07D 231/38, A61K 7/13

(54) **Bispyrazolazaverbindungen, Verfahren zu ihrer Herstellung und diese Verbindungen enthaltende Haarfärbemittel**
Bispyrazole-aza compounds, processes for their preparation, and hair dyeing agents containing these compounds
Composés bispyrazole-aza, procédé de leur préparation et agent de teinture pour cheveux contenant ces composés

(30) Priorität: 16.07.1997 DE 19730412
(43) Veröffentlichungstag der Anmeldung: 20.01.1999
(73) Patentinhaber: Wella Aktiengesellschaft, 64274 Darmstadt (DE)
(72) Erfinder: Döhling, Annelie, 64839 Münster (DE); Geibel, Wolfram Dr., 38088 Hünfeld (DE); Göttel, Otto Dr., 1723 Marly (CH)

(56) Entgegenhaltungen:
- EP-A- 0 375 977
- EP-A- 0 740 931
- EP-A- 0 769 531
- EP-A- 0 796 850
- WO-A-94/08971
- WO-A-97/42173
- CHEMICAL ABSTRACTS, vol. 85, no. 21, 22. November 1976 Columbus, Ohio, US; abstract no. 159975y, Seite 539; XP002081825

## Beschreibung

Gegenstand der Erfindung sind neue Bispyrazolazaverbindungen, ein Verfahren zu ihrer Herstellung sowie Haarfärbemittel mit einem Gehalt an diesen Verbindungen.

Neben den Haarfärbungen im Blondbereich sowie im Braun- bis Schwarzbereich besteht auf dem Haarfärbesektor eine steigender Bedarf nach kupferfarbigen und kastanienbraunen bis roten Nuancen. Diese Nachfrage kann zwar durch die Bereitstellung von Oxidationshaarfärbemitteln auf der Basis von bestimmten Entwicklersubstanzen, beispielsweise p-Aminophenol-Derivaten mit bestimmten Kupplersubstanzen, beispielsweise m-Aminophenol- oder m-Phenylendiamin-Derivaten, befriedigt werden. Es ist jedoch häufig erforderlich, daß diesen Haarfärbemitteln sowohl aus Gründen des Farbausgleichs zwischen Haaransatz und Haarspitzen sowie des besseren Deckungsvermögens als auch zur Optimierung des Färbeergebnisses sogenannte direktziehende Farbstoffe zugesetzt werden müssen.

Bei Haarfärbemitteln, die ausschließlich auf direktziehenden Farbstoffen basieren, ist für die Erzeugung von Brauntönen ebenfalls der Zusatz eines roten Farbstoffes zwingend erforderlich.

Zur Lösung dieser Aufgabe wurden in der Vergangenheit nahezu ausschließlich Nitrofarbstoffe verwendet. Es besteht jedoch weiterhin ein Bedürfnis nach neuen direktziehenden Farbstoffen, die excellente färberische Eigenschaften für den Rotbereich aufweisen.

Aus der WO 94/08971 A sind mit einer (2,4-Diamino-phenylamino)-Gruppe substituierte Pyrazolderivate sowie deren Verwendung in Haarfärbemitteln bekannt.

Es wurde nun überraschend gefunden, daß die neuen Bispyrazolazaverbindungen der allgemeinen Formel (I) als direktziehende Farbstoffe für den Rotbereich in hervorragender Weise geeignet sind.

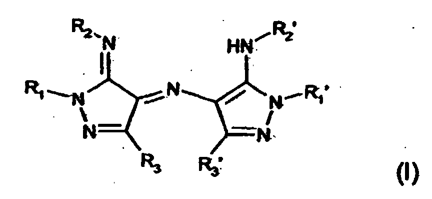

In der allgemeinen Formel (I) (die in einer ihrer möglichen tautomeren Formen dargestellt ist) können die Reste R₁, R₁', R₂, R₂', R₃ und R₃' untereinander gleich oder verschieden sein und die folgende Bedeutung haben: Wasserstoff, eine geradkettige, verzweigte oder cyclische Alkylgruppe mit bis 6 Kohlenstoffatomen, eine geradkettige oder verzweigte Monohydroxyalkylgruppe oder Polyhydroxyalkylgruppe mit 1 bis 6 Kohlenstoffatomen, eine geradkettige oder verzweigte Alkoxyalkylgruppe mit 2 bis 6 Kohlenstoffatomen, eine geradkettige oder verzweigte Monoaminoalkylgruppe oder Polyaminoalkylgruppe mit 2 bis 6 Kohlenstoffatomen oder einen aromatischen oder heteroaromatischen Ring, der mit einem oder mehreren Halogenatomen, einer C1 bis C4-Alkylgruppe, einer Nitrogruppe, einer Sulfonsäuregruppe, einer Carbonsäuregruppe oder einer C1 bis C4-Alkoxygruppe substituiert sein kann, oder eine Benzylgruppe, die mit einem oder mehreren Halogenatomen, einer Hydroxygruppe, einer C1 bis C4-Alkylgruppe, einer Nitrogruppe oder einer C1 bis C4-Alkoxygruppe substituiert sein kann.

Bevorzugte Verbindungen gemäß allgemeiner Formel (I) sind Verbindungen, in denen R₁/R₁', R₂/R₂' und R₃/R₃' unabhängig voneinander die folgende Bedeutung haben:
R₁ = R₁' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₂ = R₂' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₃ = R₃' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann.

Von den vorgenannten Verbindungen der allgemeinen Formel (I) sind insbesondere Verbindungen, in denen R₁/R₁', R₂/R₂' und R₃/R₃' unabhängig voneinander die folgende Bedeutung haben, geeignet:
R₁ = R₁' = Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₂ = R₂' = Wasserstoff, Methyl, Ethyl, Isopropyl, t-Butyl, 2-Hydroxyethyl, 2,3-Dihydroxypropyl oder Benzyl, und
R₃ = R₃' = Wasserstoff, Methyl, Ethyl, Isopropyl, t-Butyl, 2-Hydroxyethyl, 2,3-Dihydroxypropyl oder Benzyl.

Die Verbindungen der Formel (I) können sowohl als freie Basen als auch als Salze mit einer organischen oder anorganischen Säure, beispielsweise Salzsäure, Schwefelsäure, Bromwasserstoffsäure, Weinsäure, Milchsäure oder Essigsäure, insbesondere als Hydrochlorid oder Sulfat, vorliegen.

Besonders bevorzugte Verbindungen sind:
(1) 4-((5-Amino-1-methyl-1H-pyrazol-4-yl)imino)-4,5-dihydro-5-imino-1-methyl-1H-pyrazol (II) 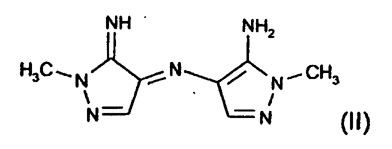
(2) 4-((5-Amino-1-isopropyl-1 H-pyrazol-4-yl)imino)-4,5-dihydro-5-imino-1-isopropyl-1H-pyrazol (III) 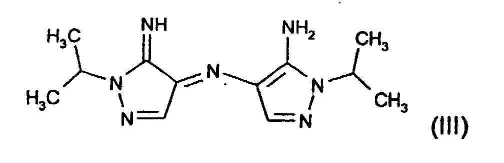
(3) 4-((5-Amino-1-(2-hydroxyethyl)-1H-pyrazol-4-yl)imino)-4,5-dihydro-1-(2-hydroxyethyl)-5-imino-1H-pyrazol-hemisulfat-hemihydrat (IV) 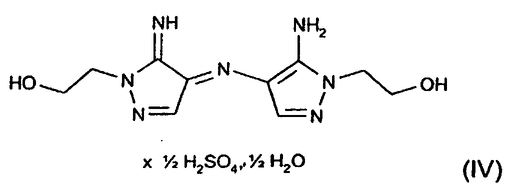
(4) 4-((5-Amino-1,3-dimethyl)-1 H-pyrazol-4-yl)imino-4,5-dihydro-1 ,3-dimethyl-5-imino-1H-pyrazol (V) 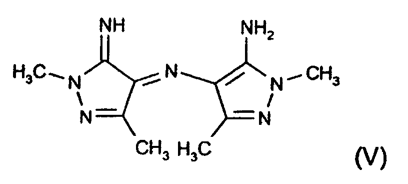
(5) 4-((5-(2-Hydroxyethyl)amino-1-methyl-1H-pyrazol-4-yl)imino)-4,5-dihydro-5-(2-hydroxyethyl)imino-1-methyl-1H-pyrazol (VI) 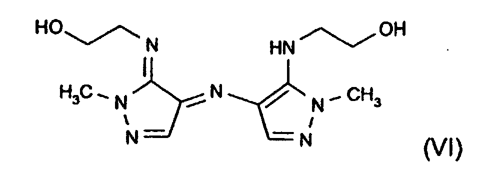
(6) 4-((5-Amino-1-*tert*.-butyl-3-methyl-1H-pyrazol-4-yl)imino)-4,5-dihydro-1-*tert*.-butyl-5-imino-3-methyl-1H-pyrazol (VIII)

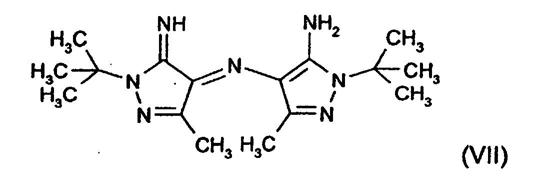

Die Verbindungen der Formel (II) bis (VII) färben Haare in leuchtend orangen bis rotvioletten Farbtönen.

Die Farbstoffe gemäß Formel (I) können durch Oxidation der entsprechend substituierten 1H-Pyrazole ((la) und (Ib)) hergestellt werden. Die Herstellung geeigneter Pyrazole erfolgt im allgemeinen durch Nitrosierung von 5-Aminopyrazolen in Position 4 und anschließender Reduktion der Nitrosogruppe zur Aminogruppe. Zur Herstellung der als Ausgangsverbindungen eingesetzten Pyrazole sei insbesondere auf die Literatur H. Dorn und H. Dilcher, Liebigs Annalen der Chemie 717, 118, (1968) und P. G. Baraldi et al., Synthesis 1984, 148 sowie die EP-OS 0 375 977 und EP-OS 0 740 931 verwiesen.

Die Herstellung der erfindungsgemäßen zweikemigen Pyrazole erfolgt vorzugsweise nach dem folgenden Reaktionsschema durch Umsetzung der Pyrazole der Formel (Ia) und (Ib), wobei die Reste R₁, R₁', R₂, R₂', R₃ und R₃' die gleiche Bedeutung wie in Formel (I) haben, mit einem Oxidationsmittel in einem neutralen oder basischen Medium, vorzugsweise einer wäßrigen, alkoholischen oder wäßrig-alkoholischen neutralen oder basischen Lösung oder Suspension:

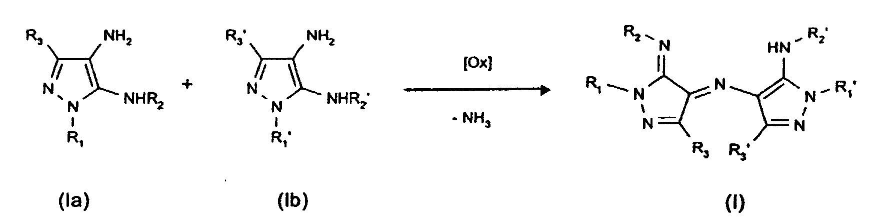

Geeignete Oxidationsmittel sind vor allem Sauerstoff, zum Beispiel in Form von Luftsauerstoff oder Wasserstoffperoxid. Andere gängige Oxidationsmittel wie beispielsweise Kaliumhexacyanoferrat, Kaliumpermanganat, Kaliumdichromat oder Natriumbromat können zwar prinzipiell ebenfalls eigesetzt werden, sie eignen sich jedoch nut bedingt; zum einen besitzen manche Oxidationsmittel eine Eigenfarbe, zum anderen bilden sie nichtflüchtige Rückstände, die bei der Produktaufarbeitung zu Farbstoff-Mischsalzen führen können. Als Basen eignen sich insbesondere Ammoniak oder Triethylamin.

Die Verbindungen der Formel (I) sind grundsätzlich sowohl zum Färben von natürlichen Fasern, wie zum Beispiel keratinischen Fasern wie Wolle und menschlichen Haaren, Baumwolle, Jute, Sisal, Leinen oder Seide, als auch modifizierten Naturfasern, wie zum Beispiel Regeneratcellulose, Nitrocellulose, Alkylcellulose oder Hydroxyalkylcellulose oder Acetylcellulose, und synthetischen Fasern, wie zum Beispiel Polyamidfasern, Polyurethanfasern oder Polyesterfasern geeignet.

Insbesondere sind die Verbindungen der Formel (I) zur Färbung von Haaren, vorzugsweise menschlichen Haaren, geeignet.

Ein weiterer Gegenstand der Erfindung ist daher neben den Verbindungen der Formel (I) ein Mittel zur Färbung von Haaren mit einem Gehalt an einer oder mehreren Verbindungen gemäß der allgemeinen Formel (I).

Der Gesamtgehalt der Verbindungen der Formel (I) beträgt in dem erfindungsgemäßen Haarfärbemittel etwa 0,01 bis 4 Gewichtsprozent, vorzugsweise 0,02 bis 2 Gewichtsprozent.

Das erfindungsgemäße Mittel kann sowohl ohne Oxidationsmittel als auch unter Zugabe eines Oxidationsmittels angewandt werden, wobei im letzteren Fall übliche Entwicklersubstanzen und Kupplersubstanzen zugesetzt werden.

Wird das Haarfärbemittel ohne Oxidationsmittelzugabe angewendet, so kann es neben den Farbstoffen der Formel (I) zusätzlich noch weitere bekannte direktfärbende Haarfarbstoffe aus der Gruppe bestehend aus Nitrofarbstoffen, Azofarbstoffen, Anthrachinonfarbstoffen und Triphenylmethanfarbstoffen, alleine oder im Gemisch miteinander, enthalten, wobei der Zusatz von Nitrofarbstoffen besonders bevorzugt ist.

Beispiele für geeignete Nitrofarbstoffe sind: 1,4-Bis[(2-hydroxyethyl)-amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydro-chlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)-amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)-amino]-4-nitrobenzol, 4-[(2,3-Dihydroxypropyl)-amino]-3-nitro-1-trifluormethyl-benzol (HC Yellow No. 6), 1-Chlor-2,4-bis-[(2-hydroxyethyl)-amino]-5-nitrobenzol (HC Yellow No. 10), 4-[(2-Hydroxyethyl)amino]-3-nitro-1-methylbenzol, 1-Chlor-4-[(2-hydroxyethyl)-amino]-3-nitrobenzol (HC Yellow No. 12), 4-[(2-Hydroxyethyl)amino]-3-nitro-1-trifluormethylbenzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitrophenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin und 7-Amino-3,4-dihydro-6-nitro-2H-1,4-benzoxazin (HC Red No. 14).

Beispiele für geeignete Azofarbstoffe sind N,N-Di(2-hydroxyethyl)-3-methyl-4-((4-nitrophenyl)azo)anilin (Cl 11210), 1-((4-Amino-3-nitrophenyl)-azo)-7-(trimethylammonium)-2-naphthol-chlorid (Basic Brown 17), 1-(2-Hydroxy-4-sulfo-6-nitro)-naphthylazo-2-naphthol (Cl 15700), 4-((4-Aminophenyl)azo)-1-[di(2-hydroxyethyl)amino]-3-methylbenzol (HC Yellow 7), 1,4-Dimethyl-5-((4-(dimethylamino)phenyl)azo)-1,2,4-triazolium-chlorid (Cl 11055), 2-Hydroxy-1-((2-methoxyphenyl)azo)-7-(trimethylammonium)-naphthalin-chlorid (CI 12245), 1-(4-Aminophenylazo)-2-hydroxy-7-trimethylammonium-naphthalin-chlorid, 4-(3-Trimethylammoniumphenylazo)-1-phenyl-3-methyl-pyrazol-5-on-chlorid (CI 12719), 4-Hydroxy-3-[(4-sulfonaphth-1-yl)azo]-naphthalin-1-sulfonsäure-dinatriumsalz (Cl 12720), 1-(4-Sulfophenylazo)-2-hydroxynaphthalin (Cl 15510), 6-Hydroxy-5-((4-sulfophenyl)azo)-2-naphthalinsulfonsäure-dinatriumsalz (CI 15985), 4-((4-Aminophenyl)azo)-1-[di(2-hydroxyethyl)amino]-benzol, 4-((4-Aminophenyl)azo)-1-[di(2-hydroxyethyl)amino]-3-methylbenzol (HC Yellow 7), 2,6-Diamino-3-((pyridin-3-yl)azo)-pyridin, 8-Amino-1-hydroxy-2-(phenylazo)-3,6-naphthalindisulfonsäure-dinatriumsalz (CI 17200), 5-Acetylamino-4-hydroxy-3-[(2-methylphenyl)azo]-2,7-naphthalindisulfonsäure-dinatriumsalz (CI 18065) und 4-((2,4-Dihydroxy-3-((2,4-dimethylphenyl)azo)phenyl)azo)-benzolsulfonsäure-natriumsalz (Cl 20170).

Beispiele für geeignete Anthrachinonfarbstoffe sind 1,4-Bis-(2,3-dihydroxypropyl)amino-anthrachinon, 1-Methylamino-4-(2-hydroxyethyl)-amino-anthrachinon, 2-(2-Aminoethyl)-amino-anthrachinon, 2-Brom-4,8-diamino-6-(3-trimethylammonium)-phenylamino-1,5-naphthochinon-chlorid (Cl 56059), 1-Hydroxy-4-((4-methyl-2-sulfophenyl)amino)-9,10-anthrachinon, 1,4-Diamino-anthrachinon, 1-Amino-4-cyclohexylamino-2-sulfo-anthrachinon, 4-Aminopropylamino-1-methylamino-anthrachinon, 1-Aminopropylamino-anthrachinon, 1,4-Diamino-2-methoxy-anthrachinon und 1,4-Dihydroxy-5,8-(bis(2-hydroxyethyl)amino)-anthrachinon.

Beispiele für geeignete Triphenylmethanfarbstoffe sind 4',4",4"'-Triamino-3-methyl-triphenylcarbeniumchlorid, Bis-(4,4-Diethylaminophenyl)-4'ethylamino-naphthyl-carbeniumchlorid, Bis-(4,4-dimethylaminophenyl)-4'phenylamino-naphthyl-carbeniumchlorid (Basic Blue 26, Cl 44045) und Bis(4-(dimethylamino)phenyl)-(3,7-disulfo-2-hydroxynaph-1-yl)carbeniumbetain-mononatriumsalz.

Die vorstehend genannten direktziehenden Nitrofarbstoffe, Azofarbstoffe, Anthrachinonfarbstoffe oder Triphenylmethanfarbstoffe können in einer Gesamtmenge von etwa 0,01 bis 4 Gewichtsprozent enthalten sein, wobei der Gesamtgehalt an nicht-oxidativen Farbstoffen vorzugsweise etwa 0,1 bis 5,0 Gewichtsprozent beträgt.

Die Zubereitungsform des erfindungsgemäßen Haarfärbemittels auf der Basis von Verbindungen der allgemeinen Formel (I) sowie gegebenenfalls direktfärbenden Farbstoffen kann beispielsweise eine Lösung, insbesondere eine wäßrig-alkoholische Lösung sein. Bevorzugte Zubereitungsformen sind weiterhin Cremes, Gele oder Emulsionen. Ebenfalls ist es möglich, diese Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder Aerosolschaum aus einem Druckbehälter zu entnehmen.

Der pH-Wert dieses Färbemittels liegt im Bereich von 3 bis 12, insbesondere bei einem pH-Wert von 8 bis 11,5, wobei die Einstellung des gewünschten alkalischen pH-Wertes vorzugsweise mit Ammoniak erfolgt, jedoch auch mit organischen Aminen wie beispielsweise Monoethanolamin oder Triethanolamin vorgenommen werden kann, während für die Einstellung eines sauren pH-Wertes je nach gewünschten pH-Wert verdünnte organische oder anorganische Säuren, wie zum Beispiel Salzsäure, Schwefelsäure, Phosphorsäure, Ascorbinsäure oder Milchsäure, verwendet werden.

Die Anwendung dieses Färbemittels erfolgt in üblicher Weise durch Aufbringen einer für die Färbung ausreichenden Menge des Mittels auf die Haare, mit denen es eine Zeit lang, vorzugsweise 15 bis 30 Minuten, in Kontakt bleibt. Anschließend wird das Haar mit Wasser, sodann gegebenenfalls noch mit einer wäßrigen Lösung einer schwachen organischen Säure gespült und abschließend getrocknet. Als schwache organische Säure können beispielsweise Essigsäure, Zitronensäure oder Weinsäure in Form einer verdünnten wäßrigen Lösung Verwendung finden.

Das vorstehend beschriebene Haarfärbemittel ohne Oxidationsmittelzugabe kann weiterhin für kosmetische Mittel übliche natürliche oder synthetische Polymere beziehungsweise modifizierte Polymere natürlichen Ursprungs enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, basische Polymerisate von Estem der Polyacrylsäure, Polymethylacrylsäure und Aminoalkoholen beispielsweise deren Salze oder Quaternisierungsprodukte, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (entacetyliertes Chitin) oder Chitosanderivate, eingesetzt werden können.

Die vorgenannten Polymere können in dem erfindungsgemäßen Mittel in der für solche Mittel üblichen Mengen, insbesondere in einer Menge von etwa 1 bis 5 Gewichtsprozent, enthalten. Der pH-Wert des erfindungsgemäßen Tönungsfestigers oder Farbfestigers beträgt etwa 6,0 bis 9,0.

Die Anwendung des Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel ohne Oxidationsmittelzugabe gegebenenfalls weitere für Haarfärbemittel übliche Zusätze, wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehören auch, wie bereits erwähnt, Haarfärbemittel, bei denen die Zugabe eines Oxidationsmittels erforderlich ist. Diese Färbemittel enthalten außer den Farbstoffen der allgemeinen Formel (I) sowie gegebenenfalls weiteren üblichen direkt auf das Haar aufziehenden Farbstoffen aus der Gruppe bestehend aus Nitrofarbstoffen, Azofarbstoffen, Anthrachinonfarbstoffen und Triphenylmethanfarbstoffen zusätzlich bekannte Oxidationsfarbstoff-Vorstufen, die einer oxidativen Behandlung bedürfen (Entwicklersubstanzen und Kupplersubstanzen).

Als Entwicklersubstanzen werden in der Regel aromatische Verbindungen aus der Gruppe der p-Phenylendiamine, p-Aminophenole und 4,5-Diaminopyrazole, beispielsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methyl-phenyl)methyl]-1H-pyrazol, 1-[(4-Chlorphenyl)methyl]-4,5-diamino-1H-pyrazol, 4,5-Diamino-1-methyl-1 H-pyrazol, 4,5-Diamino-1,3-dimethylpyrazol, 2,4-Diamino-3-*tert*.-butyl-1-(2-hydroxyethyl)-pyrazol, 2,4-Diamino-1-methyl-3-phenyl-pyrazol, 2-Amino-phenol, 2-Amino-6-methyl-phenol und 2-Amino-5-methyl-phenol, alleine oder in Kombination miteinander verwendet.

Als Kupplersubstanzen werden in der Regel m-Phenylendiamine, m-Aminophenole oder Resorcine, wie zum Beispiel 2,4-Diamino-1,5-di(2-hydroxyethoxy)benzol, N-(3-Dimethylamino-phenyl)-hamstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diaminobenzol, 2-Amino-1-(2-hydroxy-ethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxy-ethyl)amino]-phenol, 5-Amino-2-ethyl-phenol, 2-(4-Amino-2-hydroxy-phenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion alleine oder in Kombination miteinander, verwendet.

Weitere für die Haarfärbung bekannte und übliche Oxidationsfarbstoffe werden unter anderem in dem Buch von E. Sagarin, "Cosmetics, Science and Technology" (1957), Interscience Publishers Inc., New York, Seiten 503 ff, sowie in dem Buch von H. Janistyn, "Handbuch der Kosmetika und Riechstoffe, Band 3" (1973), Seite 388 bis 397, beschrieben.

Mit Kombinationen aus Oxidationsfarbstoffen und den Farbstoffen der Formel (I) lassen sich sehr gut natürliche Blondtöne und Brauntöne, aber auch modische Nuancen herstellen.

Die erfindungsgemäßen Farbstoffe der Formel (I) sind in dem Färbemittel mit Oxidationsmittel-Zugabe in einer Menge von etwa 0,01 bis 4,0 Gewichtsprozent, vorzugsweise 0,02 bis 2,0 Gewichtsprozent, enthalten, wobei der Gesamtgehalt an nicht-oxidativen Farbstoffen vorzugsweise etwa 0,1 bis 5,0 Gewichtsprozent beträgt.

Die Gesamtkonzentration an Oxidationsfarbstoff-Vorstufen beträgt in dem erfindungsgemäßen Oxidationshaarfärbemittel etwa 0,1 und 10 Gewichtsprozent, wobei eine Menge von 0,2 bis 2 Gewichtsprozent bevorzugt ist.

Das Oxidationshaarfärbemittel kann sowohl sauer oder neutral als auch basisch eingestellt sein, wobei ein pH-Wert von 6 bis 12, insbesondere ein pH-Wert von etwa 8,0 bis 11,5, bevorzugt ist. Die Einstellung des gewünschten pH-Wertes erfolgt hierbei in der Regel mit Ammoniak; es können aber auch andere organische Amine, zum Beispiel Monoethanolamin oder Triethanolamin, verwendet werden.

Als Oxidationsmittel zur Entwicklung der Haarfärbung kommen in erster Linie Wasserstoffperoxid und dessen Additionsverbindungen in Betracht. Die Zubereitungsform des erfindungsgemäßen Oxidationshaarfärbemittels kann die gleiche wie bei dem erfindungsgemäßen nicht-oxidativen Haarfärbemittel sein, wobei das Oxidationshaarfärbemittel vorzugsweise in Form einer Creme oder eines Gels vorliegt.

Übliche Zusätze in Lösungen, Cremes, Emulsionen oder Gelen sind zum Beispiel Lösungsmittel wie Wasser, niedere aliphatische einwertige oder mehrwertige Alkohole, deren Ester und Ether, beispielsweise Alkanole, insbesondere mit 1 bis 4 C-Atomen, beispielsweise Ethanol, Propanol oder Isopropanol, Butanol, Isobutanol, zweiwertige und dreiwertige Alkohole, insbesondere solche mit 2 bis 6 C-Atomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylenglycol, Tripropylenglycol, Polypropylenglycol, niedere Alkylether von mehrwertigen Alkoholen, wie Ethylenglycolmonomethylether oder Ethylenglycolmonoethylether oder Ethylenglycolmonopropylether oder Ethylenglycolmonobutylether, Diethylenglycolmonomethylether oder Diethylenglycolmonoethylether, Triethylenglycolmonomethylether oder Triethylenglycolmonoethylether, Ketone und Ketoalkohole, insbesondere solche mit 3 bis 7 C-Atomen, wie zum Beispiel Aceton, Methylethylketon, Diethylketon, Methylisobutylketon, Methylphenylketon, Cyclopentanon, Cyclohexanon und Diacetonalkohol, Ether, wie z.B. Dibutylether, Tetrahydrofuran, Dioxan, Diisopropylether, Ester wie zum Beispiel Ethylformiat, Methylformiat, Methylacetat, Ethylacetat, Propylenacetat, Butylacetat, Phenylacetat, Ethylenglycolmonoethyletheracetat und Essigsäurehydroxyethylester, Amide wie zum Beispiel Dimethylformamid und Dimethylacetamid, N-Methylpyrrolidon, sowie Harnstoff, Tetramethylharnstoff und Thiodiglycol.

Weiterhin können in dem erfindungsgemäßen Haarfärbemittel Netzmittel oder Emulgatoren aus den Klassen der anionischen, kationischen, amphoteren, nichtionogenen oder zwitterionischen oberflächenaktiven Substanzen wie Fettalkoholsulfate, Alkansulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein. Neben Wasser kann auch auch ein wasserlösliches organisches Lösungsmittel oder ein Gemisch derartiger Lösungsmittel sowie ein Wasser/Lösungsmittel-Gemisch verwendet werden.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent.

Die erfindungsgemäßen Oxidationshaarfärbemittel werden in üblicher Weise angewendet, indem man das Haarfärbemittel vor der Behandlung mit einem Oxidationsmittel vermischt und eine zur Färbung des Haares ausreichende Menge der Mischung, im allgemeinen etwa 50 bis 150 Gramm, auf das Haar aufträgt. Nach einer für die Haarfärbung ausreichenden Einwirkungszeit, die üblicherweise etwa 10 bis 45 Minuten beträgt, wird das Haar mit Wasser gespült, gegebenenfalls mit einem Shampoo gewaschen und /oder mit der wäßrigen Lösung einer schwachen organischen Säure, wie zum Beispiel Zitronensäure'oder Weinsäure, nachgespült, und sodann getrocknet.

Hinsichtlich der färberischen Möglichkeiten bietet das erfindungsgemäße Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei wird das Färbemittel je nach Zusammensetzung entweder in Verbindung mit einem Oxidationsmittel oder auch ohne Zusatz eines Oxidationsmittels angewandt.

Die nachstehenden Beispiele sollen den Erfindungsgegenstand näher erläutern, ohne ihn auf die angeführten Beispiele zu beschränken.

### Beispiele

### Synthesebeispiele

### Beispiel 1: Synthese von 4-((5-Amino-1 -methyl-1 H-pyrazol-4-yl)imino)-4,5-dihydro-5-imino-1-methyl-1H-pyrazol (II)

18,5 g 4,5-Diamino-1-methyl-1H-pyrazol-dihydrochlorid werden in 50 ml Wasser gelöst und durch Zugabe von konzentriertem Ammoniak auf einen pH-Wert von 9 eingestellt. Anschließend wird mit 17 ml 30%igem Wasserstoffperoxid versetzt. Die Lösung färbt sich spontan dunkelrot. Nach 1 Stunde wird mit konzentrierter Natronlauge auf einen pH-Wert von 12 eingestellt und der ausgefallene Farbstoff abgesaugt. Nachwaschen mit Wasser und trocknen im Vakuum ergeben 7,8 g dunkelrote Kristalle mit einem Schmelzpunkt von über 250°C (unter Zersetzung).
Massenspektrum: M⁺ = 205
¹H-NMR (DMSO-d₆): δ_{(TMS)} = 8,24 ppm (s, 2H), 3,60 ppm (s, 6H).
λₘₐₓ [H₂O] = 478 nm

### Beispiel 2: Synthese von 4-((5-Amino-1-isopropyl-1H-pyrazol-4-yl)imino)-4,5-dihydro-5-imino-1-isopropyl-1H-pyrazol (III)

23,8 g 4,5-Diamino-1-isopropyl-1H-pyrazol-sulfat werden in 240 ml Isopropanol suspendiert, mit 40,5 g Triethylamin versetzt und 24 Stunden mit einem Begasungsrührer gerührt. Innerhalb von wenigen Minuten erhält man eine dunkelrote Lösung in der nach etwa 1 Stunde das Produkt auszukristallisieren beginnt. Man filtriert den Farbstoff ab und suspendiert zur Freisetzung der Farbstoffbase 30 Minuten in einer Mischung aus 300 ml Wasser und 69,6 ml 1n Natronlauge. Absaugen und nachwaschen mit wenig Eiswasser liefert 8,8 g dunkelrote Kristalle mit einem Schmelzpunkt von 174,4°C (unter Zersetzung).
λₘₐₓ [H₂O] = 480 nm

| Elementaranalyse C₁₂H₁₉N₇ (MG = 261,33) | | | |
|---|---|---|---|
| | C | H | N |
| berechnet | 55,15% | 7,33% | 37,52% |
| gefunden | 55,45% | 7,44% | 37,18% |

### Beispiel 3: Synthese von 4-((5-Amino-1-(2-hydroxyethyl)-1H-pyrazol-4-yl)imino)-4,5-dihydro-1-(2-hydroxyethyl)-5-imino-1H-pyrazol-hemisulfathemihydrat (IV)

24 g 4,5-Diamino-1-(2-hydroxyethyl)-1H-pyrazol-sulfat werden in einer Mischung aus 30 ml Wasser und 20 ml Ethanol suspendiert. Die Suspension wird mit konzentriertem Ammoniak auf einen pH-Wert von 9 eingestellt und m 17 ml 30%igem Wasserstoffperoxid versetzt. Die Lösung färbt sich spontan dunkelrot. Nach 1 Stunde wird mit verdünnter Schwefelsäure neutralisiert und am Rotationsverdampfer auf die Hälfte des Volumens eingeengt. Rühren im Eisbad ergibt 12 g dunkelrote Kristalle mit metallischem Oberflächenglanz und einem Schmelzpunkt von 233°C.
λₘₐₓ [H₂O] = 491 nm

| Elementaranalyse: C₁₀H₁₅N₇O₂ x 0.5 H₂SO₄ x 0.5 H₂O (MG = 323,33) | | | | |
|---|---|---|---|---|
| | C | H | N | S |
| berechnet | 37,15% | 5,29% | 30,32% | 5,05% |
| gefunden | 37,25% | 5,37% | 30,37% | 5,03% |

Die Struktur dieser Verbindung wurde durch eine Röntgenstrukturanalyse bestätigt.

### Beispiel 4: Synthese von 4-((5-Amino-1,3-dimethyl)-1H-pyrazol-4-yl)imino-4,5-dihydro-1,3-dimethyl-5-imino-1H-pyrazol (V)

19,9 g 4,5-Diamino-1,3-dimethyl-1H-pyrazol-dihydrochlorid werden in 50 ml Wasser gelöst, mit konzentriertem Ammoniak auf einen pH-Wert von 9 eingestellt und mit 17 ml 30%igem Wasserstoffperoxid versetzt.
Die Lösung färbt sich spontan dunkelrot. Nach 2 Stunden wird der Farbstoff abgesaugt und mit wenig Isopropanol nachgewaschen. Das Rohprodukt besteht aus dem roten Farbstoff mit λₘₐₓ [H₂O] = 537 nm sowie einem gelben Nebenprodukt mit λₘₐₓ [H₂O] = 419 nm.

### Beispiel 5: 4-((5-(2-Hydroxyethyl)amino-1-methyl-1H-pyrazol-4-yl)imino)-4,5-dihydro-5-(2-hydroxyethyl)imino-1-methyl-1H-pyrazol (VI)

22,9 4-Amino-5-(2-hydroxyethyl)amino-1H-pyrazol-dihydrochlorid werden wie in Beispiel 4 umgesetzt. Es werden 3,7 g Rohprodukt erhalten, welches eine Verunreinigung enthält.
¹H-NMR (DMSO-*d*_{*6*}): δ_{(TMS)} = 8,50 ppm (s, breit, 3H), 8,16 ppm (s, 2H), 3,60 ppm (m, 14H).
λₘₐₓ [H₂O] = 488 nm

### Beispiel 6 4-((5-Amino-3-methyl-1-phenyl-1H-pyrazol-4-yl)imino)-4,5-dihydro-5-imino-3-methyl-1-phenyl-1H-pyrazol (VII)

2,6 g 4,5-Diamino-3-methyl-1-phenyl-1 H-pyrazol-dihydrochlorid werden in einer Mischung aus 30 ml Wasser und 30 ml Ethanol gelöst. Die erhaltene Lösung wird sodann mit konzentriertem Ammoniak auf einen pH-Wert von 9 eingestellt. Anschließend wird die Reaktionsmischung 17 Stunden lang mit einem Begasungsrührer gerührt. Der ausgefallene Farbstoff wird abgesaugt, mit Wasser gewaschen und getrocknet.
Die Ausbeute beträgt 1,2 g.
Schmelzpunkt: >250°C.

| Elementaranalyse: C₂₀H₁₉N₇ (MG = 357,42) | | | |
|---|---|---|---|
| | C | H | N |
| berechnet | 67,21% | 5,36% | 27,43% |
| gefunden | 67,13% | 5,36% | 27,33% |

### Beispiele für Haarfärbemittel

### Beispiele 7 -12: Haarfärbelösungen, basisch

Es wird folgende Färbelösung hergestellt:

| | |
|---|---|
| 10,0 g | Isopropanol |
| 10,0 g | Laurylalkohol-diglykolether-sulfat-Natriumsalz (28-prozentige wäßrige Lösung) |
| 10,0 g | Ammoniak 25-prozentige wäßrige Lösung |
| 0,3 g | Ascorbinsäure |
| X g | Farbstoff-Vorstufen und Farbstoffe gemäß Tabelle 1 |
| ad 100,0 g | Wasser vollentsalzt |

Zur Anwendung werden jeweils 10 g Haarfärbelösung mit 10 g Wasserstoffperoxid-Lösung (6-prozentige wäßrige Lösung) gemischt. Das erhaltene Färbemittel wird auf das Haar aufgetragen. Nach einer Einwirkungszeit von 20 Minuten bei 40 °C werden die Haare mit Wasser gespült, shampooniert und getrocknet.

Das erhaltene Färbeergebnis ist in der nachfolgenden Tabelle 1 zusammengefaßt.

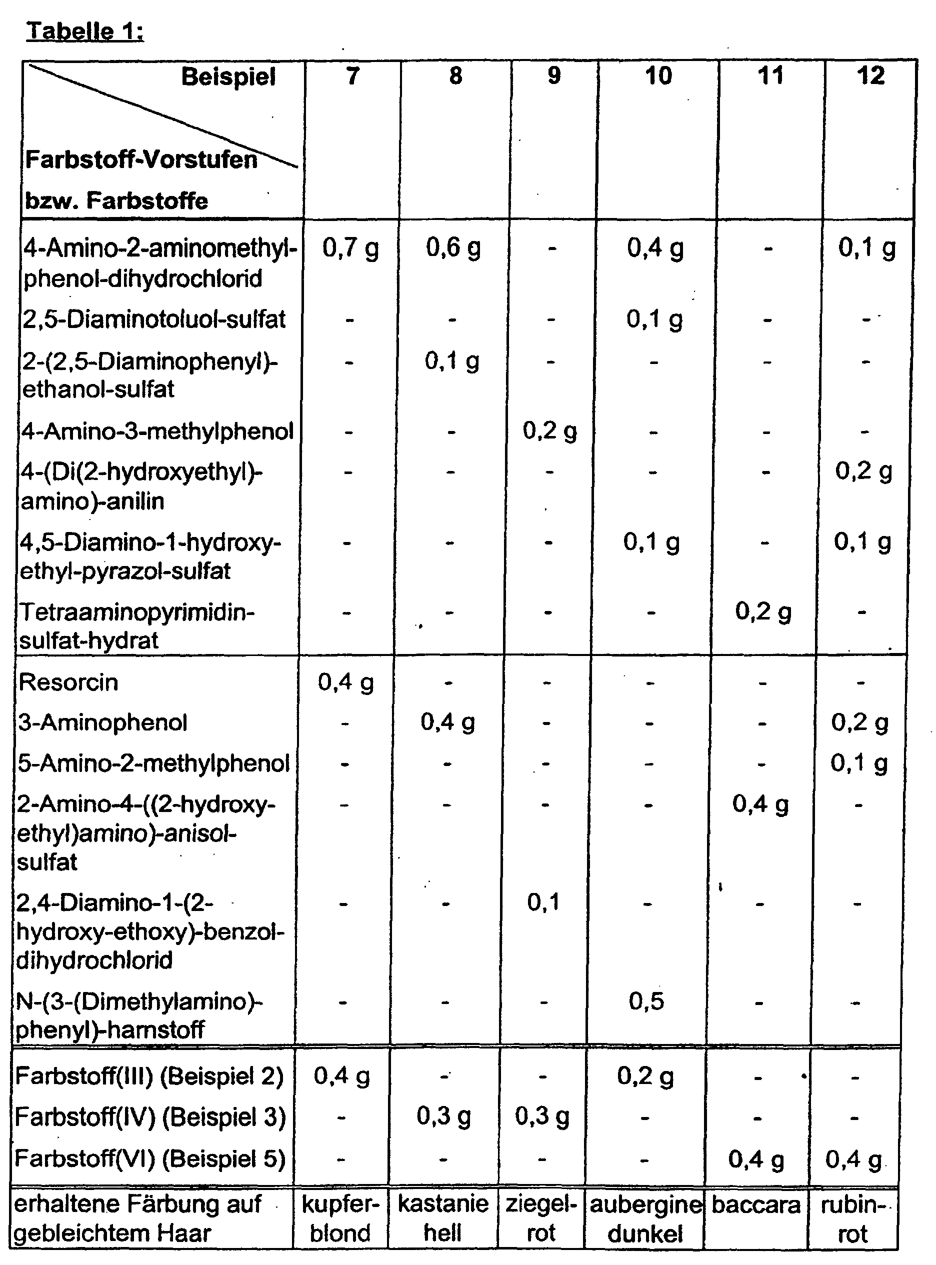

### Beispiel 13: Haarfärbemittel in Cremeform

| | |
|---|---|
| 0,40 g | 2,5-Diaminotoluol-sulfat |
| 0,60 g | 4-Amino-2-aminomethyl-phenol-dihydrochlorid |
| 0,30 g | Resorcin |
| 0,10 g | m-Aminophenol |
| 0,05 g | 4-Hydroxyindol |
| 0,10 g | 5-Amino-2-methyl-phenol |
| 0,10 g | 2-Amino-6-chlor-4-nitrophenol |
| 15,00 g | Cetylalkohol |
| 3,50 g | Natrium-Laurylalkohol-diglykolethersulfat, 28prozentige wäßrige Lösung |
| 3,00 g | Ammoniak, 25prozentige wäßrige Lösung |
| 0,30 g | Natriumsulfit, wasserfrei |
| 0,10 g | Farbstoff (V) (gemäß Beispiel 4) |
| ad 100,00 g | Wasser, entmineralisiert |

50 g dieses Haarfärbemittels werden kurz vor Gebrauch mit 50 g Wasserstoffperoxidlösung (6-prozentige wäßrige Lösung) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine kastanienbraun Färbung mit kupferrotem Reflex erhalten.

### Beispiel 14: Haarfärbemittel in Cremeform

| | |
|---|---|
| 0,30 g | 2,5-Diaminotoluol-sulfat |
| 0,20 g | 2-(2,5-Diaminophenyl)ethanol-sulfat |
| 0,04 g | 4-Amino-3-methylphenol |
| 0,10 g | 4-Amino-2-aminomethyl-phenol-dihydrochlorid |
| 0,20 g | Resorcin |
| 0,20 g | 2-Methylresorcin |
| 0,03 g | 3-Aminophenol |
| 0,07 g | 5-Amino-2-methylphenol |
| 0,02 g | 5-((2-Hydroxyethyl)amino)-2-methylphenol-hemisulfat |
| 0,10 g | 2,4-Diamino-1-fluor-5-methyl-benzol-sulfat-hydrat |
| 0,10 g | 2,4-Diamino-1,5-di(2-hydroxyethoxy)benzol |
| 0,15 g | Farbstoff (IV) (gemäß Beispiel 3) |
| 15,00 g | Cetylalkohol |
| 3,50 g | Natrium-Laurylalkohol-diglykolethersulfat, 28prozentige wäßrige Lösung |
| 3,00 g | Ammoniak, 25prozentige wäßrige Lösung |
| 0,30 g | Natriumsulfit, wasserfrei |
| 0,20 g | Ascorbinsäure |
| ad 100,00 g | Wasser, demineralisiert |

50 g dieses Haarfärbemittels werden kurz vor Gebrauch mit 50 g Wasserstoffperoxidlösung (6-prozentige wäßrige Lösung) vermischt. Anschließend trägt man das Gemisch auf blonde Naturhaare auf und läßt es 20 Minuten lang bei 40 Grad Celsius einwirken. Danach wird das Haar mit Wasser gespült und getrocknet. Das Haar hat eine anthrazit-farbene Grundfärbung mit Auberginereflex erhalten.

### Beispiel 15: Haarfärbemittel in Gelform

| | |
|---|---|
| 0,50 g | 2,5-Diaminophenylethanol-sulfat |
| 0,30 g | 3-Dimethylamino-phenylhamstoff |
| 0,10 g | 1-Naphthol |
| 0,40 g | Natriumhydroxid, fest |
| 0,50 g | Ascorbinsäure |
| 7,00 g | Isopropanol |
| 3,00 g | Glycerin |
| 15,00 g | Ölsäure |
| 10,00 g | Ammoniak, 25prozentige wäßrige Lösung |
| 0,10 g | Farbstoff (III) (gemäß Beispiel 2) |
| ad 100,00 g | Wasser, entmineralisiert |

Man vermischt kurz vor dem Gebrauch 50 g dieses Haarfärbemittels mit 50 g Wasserstoffperoxidlösung (6-prozentige wäßrige Lösung) und läßt das Gemisch 30 Minuten lang auf blonde menschliche Haare einwirken. Danach wird mit Wasser gespült und sodann getrocknet. Man erhält eine Färbung in einem intensiven Aubergineton.

### Beispiel 16: Haarfärbelösung, sauer

| | |
|---|---|
| 0,60 g | 4-Amino-2-aminomethylphenol-dihydrochlorid |
| 0,10 g | Resorcin |
| 0,30 g | 4-Chlorresorcin |
| 0,30 g | Ascorbinsäure |
| 0,30 g | Natriumlaurylethersulfat |
| 1,10 g | Ammoniak, 25prozentige wäßrige Lösung |
| 0,10 g | Farbstoff (II) (gemäß Beispiel 1) |
| ad 100,00 g | Wasser, entmineralisiert |

Der pH-Wert der Farbträgermasse wird falls erforderlich mit einer verdünnten Ammoniaklösung oder verdünnter Salzsäure auf einen pH-Wert von 6,8 eingestellt.

Unmittelbar vor der Anwendung werden 20 Gramm der Haarfärbelösung mit 20 Gramm einer 6-prozentigen wäßrigen Lösung Wasserstoffperoxidlösung (pH = 6,8) vermischt und das erhaltene gebrauchsfertige Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.
Das so behandelte Haar ist in einem helleren Kupferton gefärbt.

### Beispiel 17: Haarfärbelösung, nicht-oxidativ

| | |
|---|---|
| 0,05 g | Basic Blue 7 (C.I. 42595) |
| 0,03 g | 1-Methylammo-4-[N-methyl-N-(2,3-dihydroxypropyl)amino]-2-nitrobenzol |
| 0,15 g | N-(2,3-Dihydroxypropyl)-2-nitro-4-trifluormethyl-anilin |
| 0,15 g | Farbstoff (IV) (gemäß Beispiel 3) |
| ad 100,00 g | Wasser, entmineralisiert |

Der pH-Wert der Farbträgermasse wird falls erforderlich mit einer verdünnten Ammoniaklösung oder verdünnter Salzsäure auf einen pH-Wert von 6,8 eingestellt.

Das erhaltene Färbemittel (pH = 6,8) wird auf gebleichte Haare aufgetragen. Nach einer Einwirkungszeit von 20 Minuten bei 40°C werden die Haare mit Wasser gespült und getrocknet. Die Haare sind in einem kastanienbraunen Ton gefärbt.

### Bespiele 18 - 20: Haarfärbemittel, nicht-oxidativ

Es wird folgende Lösung hergestellt:

| | |
|---|---|
| 10,0 g | Isopropanol |
| 1,0 g | Hydroxyethylcellulose |
| X g | Farbstoffe gemäß Tabelle 2 |
| ad 100,0 g | Wasser demineralisiert |

Der in der Tabelle 2 angegebene pH-Wert der Haarfärbemittel wird durch Zusatz von Citronensäure oder Natronlauge eingestellt.

Die erhaltenen Färbungen sind in der nachfolgenden Tabelle 2 zusammengefaßt.

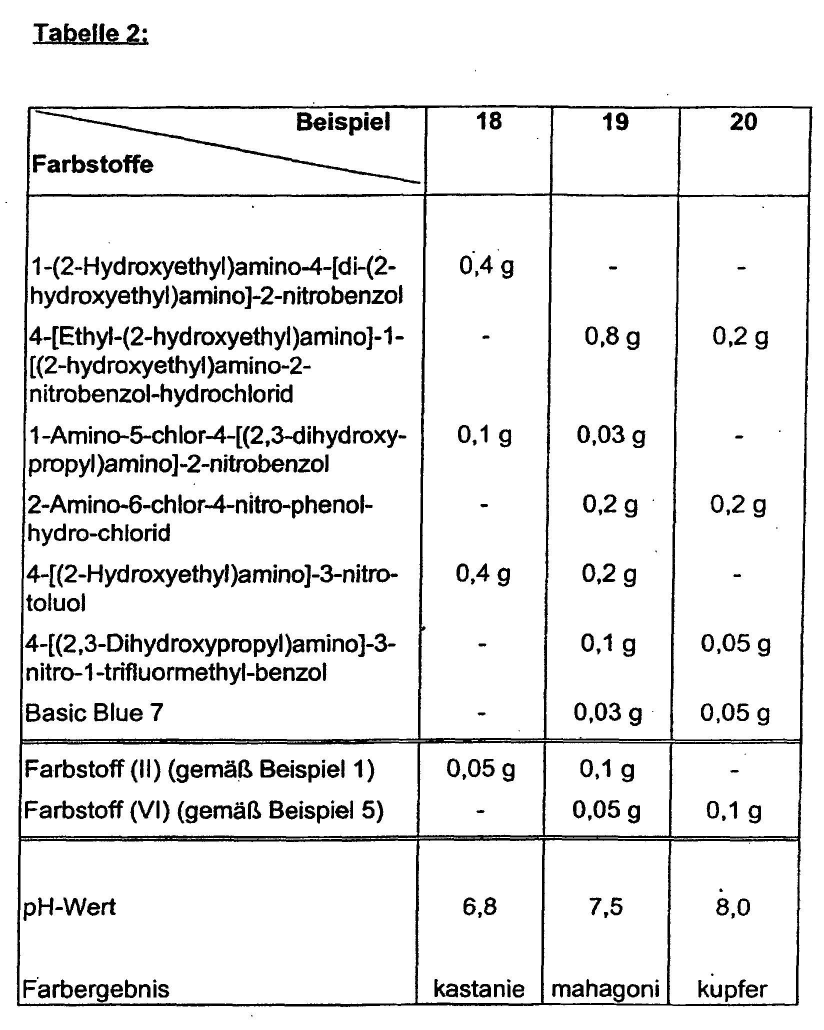

### Beispiel 21: Haarfärbemittel mit festigender Wirkung

| | |
|---|---|
| 2,00 g | Polyvinylpyrrolidon |
| 0,10 g | Glycein |
| 40,00 g | Ethanol |
| 0,15 g | N-(2,3-Dihydroxypropyl)-2-nitro-4-trifluormethyl-anilin |
| 0,05 g | Farbstoff (III) (gemäß Beispiel 2) |
| ad 100,00 g | Wasser, entmineralisiert |

Weiße menschliche Haare werden mit der vorgenannten Farbfestigerlösung befeuchtet, zur Frisur eingelegt und getrocknet. Das so behandelte Haar erhält einen rötlichen Schimmer und ist gefestigt.

## Patentansprüche

1. Bispyrazolazaverbindungen der allgemeinen Formel (I) 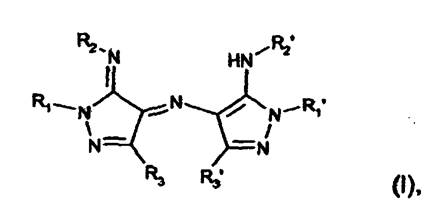 in der R₁, R₁', R₂, R₂', R₃ und R₃' gleich oder verschieden sein können und die folgende Bedeutung haben: Wasserstoff, eine geradkettige, verzweigte oder cyclische Alkylgruppe mit 1 bis 6 Kohlenstoffatomen, eine geradkettige oder verzweigte Monohydroxyalkylgruppe oder Polyhydroxyalkylgruppe mit 1 bis 6 Kohlenstoffatomen, eine geradkettige oder verzweigte Alkoxyalkylgruppe mit 2 bis 6 Kohlenstoffatomen, eine geradkettige oder verzweigte Monoaminoalkylgruppe oder Polyaminoalkylgruppe mit 2 bis 6 Kohlenstoffatomen oder einen aromatischen oder heteroaromatischen Ring, der mit einem oder mehreren Halogenatomen, einer C1 bis C4-Alkylgruppe, einer Nitrogruppe, einer Sulfonsäuregruppe, einer Carbonsäuregruppe oder einer C1 bis C4-Alkoxygruppe substituiert sein kann, oder eine Benzylgruppe, die mit einem oder mehreren Halogenatomen, einer Hydroxygruppe, einer C1 bis C4-Alkylgruppe, einer Nitrogruppe oder einer C1 bis C4-Alkoxygruppe substituiert sein kann, oder deren Salze mit organischen oder anorganischen Säuren.

2. Verbindungen nach Anspruch 1, **dadurch gekennzeichnet, daß** gilt:
R₁ = R₁' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₂ = R₂' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₃ = R₃' = Wasserstoff, Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann.

3. Verbindungen nach Anspruch 1 oder 2, **dadurch gekennzeichnet, daß** gilt:
R₁ = R₁' = Methyl, Ethyl, Isopropyl, t-Butyl, 2-Hydroxyethyl, 2,3-Dihydroxypropyl, Phenyl oder Benzyl, wobei der Phenyl- oder Benzylrest unsubstituiert oder mit einem oder mehreren Halogenatomen, einer C1 bis C4-Alkylgruppe, einer Nitrogruppe oder einer C1 bis C4-Alkoxygruppe substituiert sein kann, und
R₂ = R₂' = Wasserstoff, Methyl, Ethyl, Isopropyl, t-Butyl, 2-Hydroxyethyl, 2,3-Dihydroxypropyl oder Benzyl, und
R₃ = R₃' = Wasserstoff, Methyl, Ethyl, Isopropyl, t-Butyl, 2-Hydroxyethyl, 2,3-Dihydroxypropyl oder Benzyl.

4. Verbindungen nach einem der Ansprüche 1 bis 3, **dadurch gekennzeichnet, daß** die Verbindung der Formel (I) ausgewählt ist aus 4-((5-Amino-1-methyl-1H-pyrazol-4-yl)imino)-4,5-dihydro-5-imino-1-methyl-1 H-pyrazol, 4-((5-Amino-1-isopropyl-1H-pyrazol-4-yl)imino)-4,5-dihydro-5-imino-1 -isopropyl-1 H-pyrazol, 4-((5-Amino-1-(2-hydroxyethyl)-1H-pyrazol-4-yl)imino)-4,5-dihydro-1-(2-hydroxyethyl)-5-imino-1H-pyrazol-hemisulfathemihydrat, 4-((5-Amino-1-methyl-1H-pyrazol-4-yl)imino)-4,5-dihydro-1,3-dimethyl-5-imino-1H-pyrazol, 4-((5-(2-Hydroxyethyl)amino-1-methyl-1 H-pyrazol-4-yl)imino)-4,5-dihydro-5-(2-hydroxyethyl)imino-1-methyl-1Hpyrazol und 4-((5-Amino-1-*tert*.-butyl-3-methyl-1H-pyrazol-4-yl)imino)-4,5-dihydro-1-*tert*.-butyl-5-imino -3-methy(-1H-pyrazol.

5. Mittel zum Färben von Haaren, **dadurch gekennzeichnet, daß** es mindestens eine Bispyrazolazaverbindung nach einem der Ansprüche 1 bis 4 enthält.

6. Mittel nach Anspruch 5, **dadurch gekennzeichnet, daß** es zusätzlich Oxidationsfarbstoff-Vorstufen, die einer oxidativen Behandlung bedürfen (Entwicklersubstanzen und Kupplersubstanzen) enthält.

7. Mittel nach Anspruch 6, **dadurch gekennzeichnet, daß** die Entwicklersubstanzen ausgewählt sind aus aromatischen Verbindungen aus der Gruppe der p-Phenylendiamine, p-Aminophenole und 4,5-Diaminopyrazole und die Kupplersubstanzen ausgewählt sind aus m-Phenylendiaminen, m-Aminophenolen und Resorcinen.

8. Mittel nach einem der Ansprüche 5 bis 7, **dadurch gekennzeichnet, daß** es zusätzlich mindestens einen direktziehenden Farbstoff aus der Gruppe bestehend aus Nitrofarbstoffen, Azofarbstoffen, Anthrachinonfarbstoffen oder Triphenylmethanfarbstoffen enthält

9. Mittel nach einem der Ansprüche 5 bis 8, **dadurch gekennzeichnet**, die Verbindung der Formel (I) in einer Menge von 0,01 bis 4 Gewichtsprozent enthalten ist.

10. Mittel nach einem der Ansprüche 5, 8 und 9, **dadurch gekennzeichnet, daß** es zusätzlich natürliche oder synthetische Polymere beziehungsweise modifizierte Polymere natürlichen Ursprungs enthält.

11. Verfahren zur Herstellung der Bispyrazolazaverbindungen der Formel (I), **dadurch gekennzeichnet, daß** die Pyrazole der Formel (la) und (Ib), wobei die Reste R₁, R₁', R₂, R₂', R₃ und R₃' die gleiche Bedeutung wie in Formel (I) haben, 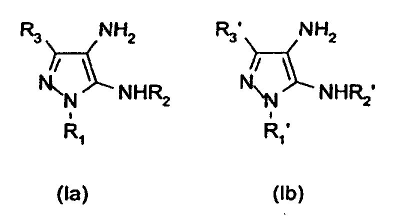 in einem neutralen oder basischen Medium mit einem Oxidationsmittel behandelt werden.

12. Verfahren nach Anspruch 11, **dadurch gekennzeichnet, daß** als Oxidationsmittel Wasserstoffperoxid oder Luftsauerstoff verwendet wird.

## Claims

1. Bispyrazole-aza compounds of the general formula (I) 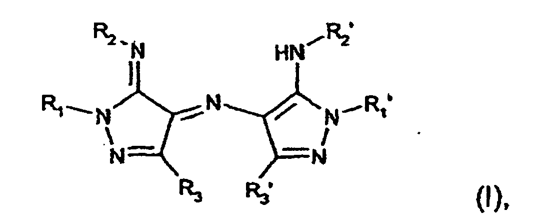 in which R₁, R₁', R₂, R₂', R₃ and R₃' may be identical or different and have the following meanings: hydrogen, a straight-chain, branched or cyclic alkyl group having 1 to 6 carbon atoms, a straight-chain or branched monohydroxyalkyl group or polyhydroxyalkyl group having 1 to 6 carbon atoms, a straight-chain or branched alkoxyalkyl group having 2 to 6 carbon atoms, a straight-chain or branched monoaminoalkyl group or polyaminoalkyl group having 2 to 6 carbon atoms or an aromatic or heteroaromatic ring, which may be substituted by one or more halogen atoms, a C1 to C4-alkyl group, a nitro group, a sulphonic acid group, a carboxylic acid group or a C1 to C4-alkoxy group, or a benzyl group, which may be substituted by one or more halogen atoms, a hydroxyl group, a C1 to C4-alkyl group, a nitro group or a C1 to C4-alkoxy group, or salts thereof with organic or inorganic acids.

2. Compounds according to Claim 1, **characterized in that** the following apply:
R₁ = R₁' = hydrogen, methyl, ethyl, isopropyl, t-butyl, 2-hydroxyethyl, 2,3-dihydroxypropyl, phenyl or benzyl, where the phenyl or benzyl radical may be unsubstituted or substituted by one or more halogen atoms, a C1 to C4-alkyl group, a nitro group or a C1 to C4-alkoxy group, and
R₂ = R₂' = hydrogen, methyl, ethyl, isopropyl, t-butyl, 2-hydroxyethyl, 2,3-dihydroxypropyl, phenyl or benzyl, where the phenyl or benzyl radical may be unsubstituted or substituted by one or more halogen atoms, a C1 to C4-alkyl group, a nitro group or a C1 to C4-alkoxy group, and
R₃ = R₃' = hydrogen, methyl, ethyl, isopropyl, t-butyl, 2-hydroxyethyl, 2,3-dihydroxypropyl, phenyl or benzyl, where the phenyl or benzyl radical may be unsubstituted or substituted by one or more halogen atoms, a C1 to C4-alkyl group, a nitro group or a C1 to C4-alkoxy group.

3. Compounds according to Claim 1 or 2, **characterized in that** the following apply:
R₁ = R₁' = methyl, ethyl, isopropyl, t-butyl, 2-hydroxyethyl, 2,3-dihydroxypropyl, phenyl or benzyl, where the phenyl or benzyl radical may be unsubstituted or substituted by one or more halogen atoms, a C1 to C4-alkyl group, a nitro group or a C1 to C4-alkoxy group, and
R₂ = R₂' = hydrogen, methyl, ethyl, isopropyl, t-butyl, 2-hydroxyethyl, 2,3-dihydroxypropyl or benzyl, and
R₃ = R₃' = hydrogen, methyl, ethyl, isopropyl, t-butyl, 2-hydroxyethyl, 2,3-dihydroxypropyl or benzyl.

4. Compounds according to one of Claims 1 to 3, **characterized in that** the compound of the formula (I) is chosen from 4-((5-amino-1-methyl-1H-pyrazol-4-yl)imino) -4,5-dihydro-5-imino-1-methyl-1H-pyrazole, 4-((5-amino-1-isopropyl-1H-pyrazol-4-yl) imino)-4, 5-dihydro-5-imino-1-isopropyl-1H-pyrazole, 4-((5-amino-1-(2-hydroxyethyl)-1H-pyrazol-4-yl)imino)-4,5-dihydro-1-(2-hydroxyethyl)-5-imino-1H-pyrazole hemisulphate hemihydrate, 4-((5-amino-1,3-dimethyl)-1H-pyrazol-4-yl)imino-4,5-dihydro-1,3-dimethyl-5-imino-1H-pyrazole, 4-((5-(2-hydroxyethyl)amino-1-methyl-1H-pyrazol-4-yl)imino)-4,5-dihydro-5-(2-hydroxyethyl)imino-1-methyl-1H-pyrazole and 4-((5-amino-1-*tert*-butyl-3-methyl-1H-pyrazol-4-yl)imino)-4,5-dihydro-1-*tert*-butyl-5-imino-3-methyl-1H-pyrazole.

5. Agent for colouring hair, **characterized in that** it comprises at least one bispyrazole-aza compound according to one of Claims 1 to 4.

6. Agent according to Claim 5, **characterized in that** it additionally comprises oxidation dye precursors which require oxidative treatment (developer substances and coupler substances).

7. Agent according to Claim 6, **characterized in that** the developer substances are chosen from aromatic compounds from the group of p-phenylenediamines, p-aminophenols and 4,5-diaminopyrazoles and the coupler substances are chosen from m-phenylene-diamines, m-aminophenols and resorcinols.

8. Agent according to one of Claims 5 to 7, **characterized in that** it additionally comprises at least one direct dye from the group consisting of nitro dyes, azo dyes, anthraquinone dyes or triphenylmethane dyes.

9. Agent according to one of Claims 5 to 8, **characterized in that** the compound of the formula (I) is present in an amount of from 0.01 to 4% by weight.

10. Agent according to one of Claims 5, 8 and 9, **characterized in that** it additionally comprises natural or synthetic polymers or modified polymers of natural origin.

11. Process for the preparation of the bispyrazole-aza compounds of the formula (I), **characterized in that** the pyrazoles of the formula (Ia) and (Ib), where the radicals R₁, R₁', R₂, R₂', R₃ and R₃' have the same meanings as in formula (I), 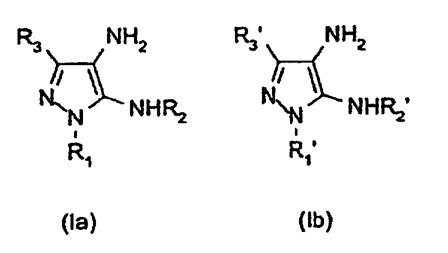 are treated with an oxidizing agent in a neutral or basic medium.

12. Process according to Claim 11, **characterized in that** the oxidizing agent used is hydrogen peroxide or atmospheric oxygen.

## Revendications

1. Composés de bispyrazolaza de formule générale (I) 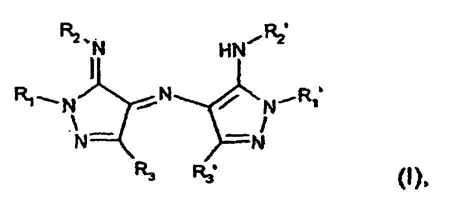 dans laquelle R₁, R₁', R₂, R₂', R₃ et R₃' peuvent être identiques ou différents et représentent l'hydrogène, un groupe alkyle à chaîne linéaire, ramifiée ou cyclique qui compte de 1 à 6 atomes de carbone, un groupe monohydroxyalkyle ou polyhydroxyalkyle, à chaîne linéaire ou ramifiée, qui compte de 1 à 6 atomes de carbone, un groupe alkoxyalkyle à chaîne linéaire ou ramifiée qui compte de 2 à 6 atomes de carbone, un groupe monoaminoalkyle ou polyaminoalkyle, à chaîne linéaire ou ramifiée, qui compte de 2 à 6 atomes de carbone ou un cycle aromatique ou hétéroaromatique qui peut être substitué avec un ou plusieurs atomes d'halogène, un groupe alkyle en C₁ à C₄, un groupe nitro, un groupe acide sulfonique, un groupe acide carboxylique ou un groupe alkoxy en C₁ à C₄, ou un groupe benzyle qui peut être substitué avec un ou plusieurs atomes d'halogène, un groupe hydroxyle, un groupe alkyle en C₁ à C₄, un groupe nitro ou un groupe alkoxy en C₁ à C₄, ou leurs sels avec des acides organiques ou minéraux.

2. Composés selon la revendication 1, **caractérisés en ce que** R₁ = R₁' = l'hydrogène, le méthyle, l'éthyle, l'isopropyle, le t-butyle, le 2-hydroxyéthyle, le 2,3-dihydroxypropyle, le phényle ou le benzyle, le groupe phényle ou benzyle pouvant être non substitué ou substitué avec un ou plusieurs atomes d'halogène, un groupe alkyle en C₁ à C₄, un groupe nitro ou un groupe alkoxy en C₁ à C₄, et
R₂ = R₂' = l'hydrogène, le méthyle, l'éthyle, l'isopropyle, le t-butyle, le 2-hydroxyéthyle, le 2,3-dihydroxypropyle, le phényle ou le benzyle, le groupe phényle ou benzyle pouvant être non substitué ou substitué avec un ou plusieurs atomes d'halogène, un groupe alkyle en C₁ à C₄, un groupe nitro ou un groupe alkoxy en C₁ à C₄, et
R₃ = R₃' = l'hydrogène, le méthyle, l'éthyle, l'isopropyle, le t-butyle, le 2-hydroxyéthyle, le 2,3-dihydroxypropyle, le phényle ou le benzyle, le groupe phényle ou benzyle pouvant être non substitué ou substitué avec un ou plusieurs atomes d'halogène, un groupe alkyle en C₁ à C₄, un groupe nitro ou un groupe alkoxy en C₁ à C₄.

3. Composés selon la revendication 1 ou 2, **caractérisés en ce que**:
R₁ = R₁' = le méthyle, l'éthyle, l'isopropyle, le t-butyle, le 2-hydroxyéthyle, le 2,3-dihydroxypropyle, le phényle ou le benzyle, le groupe phényle ou benzyle pouvant être non substitué ou substitué avec un ou plusieurs atomes d'halogène, un groupe alkyle en C₁ à C₄, un groupe nitro ou un groupe alkoxy en C₁ à C₄, et
R₂ = R₂' = l'hydrogène, le méthyle, l'éthyle, l'isopropyle, le t-butyle, le 2-hydroxyéthyle, le 2,3-dihydroxypropyle ou le benzyle, et
R₃ = R₃' = l'hydrogène, le méthyle, l'éthyle, l'isopropyle, le t-butyle, le 2-hydroxyéthyle, le 2,3-dihydroxypropyle ou le benzyle.

4. Composés selon l'une quelconque des revendications 1 à 3, **caractérisés en ce que** le composé de formule (I) est sélectionné parmi le 4-((5-amino-1-méthyl-1H-pyrazol-4-yl)imino)-4,5-dihydro-5-imino-1-méthyl-1H-pyrazole, le 4-((5-amino-1-isopropyl-1H-pyrazol-4-yl)imino)-4,5-dihydro-5-imino-1-isopropyl-1H-pyrazole, le 4-((5-amino-1-(2-hydroxyéthyl)-1H-pyrazol-4-yl)imino-4,5-dihydro-1-(2-hydroxyéthyl)-5-imino-1H-pyrazol-hémisulfate-hémihydrate, le 4-((5-amino-1,3-diméthyl)1H-pyrazol-4-yl)imino-4,5-dihydro-1,3-diméthyl-5-imino-1H-pyrazole, le 4-((5-(2-hydroxyéthyl)amino-1-méthyl-1H-pyrazol-4-yl)imino)-4,5-dihydro-5-(2-hydroxyéthyl)imino-1-méthyl-1H-pyrazole et le 4-((5-amino-1-tert-butyl-3-méthyl-1H-pyrazol-4-yl)imino)-4,5-dihydro-1-tert-butyl-5-imino-3-méthyl-1H-pyrazole.

5. Agent de coloration des cheveux, **caractérisé en ce qu'**il contient au moins un composé bispyrazolaza selon l'une quelconque des revendications 1 à 4.

6. Agent selon la revendication 5, **caractérisé en ce qu'**il contient en outre des précurseurs de colorant à oxydation qui nécessitent un traitement par oxydation (substances de développement et substances de couplage).

7. Agent selon la revendication 6, **caractérisé en ce que** les substances de développement sont sélectionnées parmi des composés aromatiques du groupe des p-phénylènediamines, des p-aminophénols et des 4,5-diaminopyrazoles, et **en ce que** les substances de couplage sont sélectionnées parmi les m-phénylènediamines, les m-aminophénols et les résorcines.

8. Agent selon l'une quelconque des revendications 5 à 7, **caractérisé en ce qu'**il contient en outre au moins un colorant à action directe sélectionné dans le groupe constitué des colorants nitro, des colorants azo, des colorants à l'anthraquinone ou des colorants à base de triphénylméthane.

9. Agent selon l'une quelconque des revendications 5 à 8, **caractérisé en ce que** le composé de formule (I) y est contenu en une quantité de 0,01 à 4 pour-cent en poids.

10. Agent selon l'une quelconque des revendications 5, 8 et 9, **caractérisé en ce qu'**il contient en outre des polymères naturels ou synthétiques ou des polymères d'origine naturelle modifiés.

11. Procédé pour la préparation des composés de bispyrazolaza de formule (I), **caractérisé en ce que** les pyrazoles de formule (Ia) et (Ib), dans lesquelles les groupes R₁, R₁', R₂, R₂', R₃ et R₃' ont la même signification que dans la formule (I) 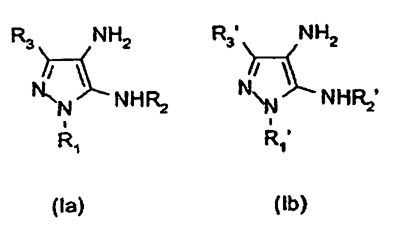 sont traités par un agent d'oxydation dans un milieu neutre ou basique.

12. Procédé selon la revendication 11, **caractérisé en ce que** l'on utilise comme agent d'oxydation du peroxyde d'hydrogène ou l'oxygène de l'air.
